Europäisches Patentamt

**European Patent Office**

**Office européen des brevets**

(11) Veröffentlichungsnummer: **0 082 395**
**A2**

(12) # EUROPÄISCHE PATENTANMELDUNG

(21) Anmeldenummer: **82111288.5**

(22) Anmeldetag: **06.12.82**

(51) Int. Cl.³: **C 12 N 9/62**
**C 12 N 1/14, A 23 K 1/165**
**//C12R1/685**

(30) Priorität: **14.12.81 DE 3149457**

(43) Veröffentlichungstag der Anmeldung:
**29.06.83 Patentblatt 83/26**

(84) Benannte Vertragsstaaten:
**AT BE CH DE FR GB IT LI LU NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Bartnik, Friedhelm, Dr.**
**Melanchtonstrasse 11**
**D-4000 Düsseldorf(DE)**

(72) Erfinder: **Schindler, Joachim, Dr.**
**Am Eichelkamp 158**
**D-4010 Hilden(DE)**

(72) Erfinder: **Weiss, Albrecht, Dr.**
**Amselweg 8**
**D-4006 Erkrath(DE)**

(72) Erfinder: **Schmid, Rolf, Dr.**
**Am Nettchesfeld 30**
**D-4000 Düsseldorf 13(DE)**

(54) **Verfahren zur Herstellung saurer Protease und diese bildende Mutanten von Pilzen der Gattung Aspergillus.**

(57) Saure Proteasen mit breitem Wirkungsspektrum werden in hohen Ausbeuten von selektierten Aspergillus-Mutanten erhalten.

Für die Selektion geht man von einem Wildstamm aus, bevorzugt Aspergillus niger var. Thienhem CBS 319.81, der extrazelluläre saure Protease bildet. Die Mutierung erfolgt vorzugsweise durch UV-Bestrahlung. Mutanten mit erhöhter proteolytischer Aktivität lassen sich selektieren, indem man sie auf Caseinatagarplatten ausspatelt, dem Agarmedium einen Inhibitor für Carboxylprotease, bevorzugt Pepstatin, zugibt, bebrütet und Mutantenkolonien mit verstärkter caseinolytischer Hofbildung isoliert.

Beansprucht werden die Mutanten Aspergillus niger AP 114-III-69 bzw. -IV-70, -74, und -80 mit den Hinterlegungs-nummern CBS 320.81; 321.81; 322.81 und 323.81, sowie der aus Boden isolierte Wildstamm Aspergillus niger var. Tien-hem AP 114 (CBS 319.81) sowie die Verwendung der daraus gewonnen Protease als Tierfutterzusatz.

4000 Düsseldorf

Henkel KGaA
ZR-FE/Patente

9. Dezember 1981

Dr. Ba/Bö.

P a t e n t a n m e l d u n g

D 5958 EP

Verfahren zur Herstellung saurer Protease und diese
bildende Mutanten von Pilzen der Gattung Aspergillus

Die Erfindung betrifft ein Verfahren zur Herstellung
saurer Proteasen mit breitem pH-Wirkungsbereich in hoher
Ausbeute von Aspergillus sowie diese bildende Mutanten.

Saure Proteasen aus Aspergillus sowie Mutanten, die die
Gewinnung dieser Proteasen in industriellem Maßstabe ermöglichen, sind bekannt. Die Erfindung hat eine Steigerung der Proteaseausbeute über das bisher bekannte Maß
hinaus zum Ziel. Dies wird erreicht durch Mutierung geeigneter Wildstämme und Auslesen von Mutanten mit erhöhter Proteasebildung.

Erfindungsgemäß geht man dabei von einem Wildstamm der
Gattung Aspergillus, vorzugsweise von Aspergillus niger
var. Tienhem CBS 319.81 aus, der aus Erdproben isoliert
wird. Dieser Stamm bildet eine extrazelluläre saure Protease, die in einem breitem pH-Bereich wirksam ist. Die
Mutierung erfolgt vorzugsweise durch UV-Bestrahlung. Die
Mutationsbehandlung wird mit den selektierten Stämmen
gegebenenfalls ein- oder mehrfach wiederholt.

Gegenstand der Erfindung ist somit ein Verfahren zur
Herstellung von saurer Protease mit breitem pH-Wirkungsbereich in hoher Ausbeute durch aerobes Züchten eines
Pilzes der Gattung Aspergillus, dadurch gekennzeichnet,
daß man

a) einen Wildstamm der Gattung Aspergillus, welcher saure Protease bildet, isoliert und

b) einer Mutierung, vorzugsweise durch UV-Bestrahlung, unterwirft und

c) Mutanten mit einer erhöhten Proteasebildung von über 15 m TU/ml selektiert, indem man sie auf Caseinatagarplatten ausspatelt, dem Agar-Medium einen Inhibitor für Carboxylprotease, vorzugsweise Pepstatin, zu gibt, einige Tage bebrütet und die Mutantenkolonien mitverstärkter caseinolytischer Hofbildung isoliert und

d) diese Mutanten, in einem Nährmedium, das assimilierbare Kohlenstoff- und Stickstoffquellen enthält, bei pH-Bereichen zwischen 3 bis 7 und Temperaturen zwischen25 und 50 $^{o}$ C züchtet und

e) die gebildete Protease abtrennt.

Gegenstand der Erfindung sind auch der Wildstamm mit der Hinterlegungsbezeichnung AP 114 (CBS 319.81) sowie die auf dem vorgenannten Wege isolierten Mutanten des Wildstamms, sofern sie in Schüttelkulturen unter den nachstehend angegebenen Bedingungen eine proteolytische Aktivität von mehr als 15 mTU/ml bilden und sofern die gebildete Protease aufgrund der nachstehend beschriebenen Tests (Isoelektrofokussierung und immunologische Prüfung) mit derjenigen des Wildstammes identisch ist. Insbesondere werden die folgenden Mutanten beansprucht:

Aspergillus niger AP 114 - III - 69    (CBS 320.81)
Aspergillus niger AP 114 -  IV - 70    (CBS 321.81)
Aspergillus niger AP 114 -  IV - 74    (CBS 322.81)
Aspergillus niger AP 114 -  IV - 80    (CBS 323.81).

Die Isolierung der Mutanten erfolgte in folgender Weise:

Auf Würzebouillonager versporte, 6 Tage alte Schrägkulturen des Wildstammes wurden mit steriler 0,005 %iger
Natriumlaurylsulfatlösung abgeschwemmt, die Sporensuspension durch eine sterile Glasfritte (D-3) zur Abtrennung der Myzelfragmente filtriert und anschließend
15 Minuten bei 6 000 U/Min. zentrifugiert. Die sedimentierten Sporen wurden in sterilem 0,1 M Acetatpuffer (pH
4,5) aufgenommen und die Sporenkonzentration unter dem
Mikroskop auf ca. $10^8$ Keime/ml eingestellt. Die eingestellte Sporenlösung wurde anschließend mit einer UV-
Lampe (Wellenlänge 254 nm, 13 Watt) bestrahlt bis zu
einer Abtötungsrate von 99,9 %. Zur Isolierung aktiver
Mutantenstämme wurden die bestrahlten Sporen auf Caseinatagarplatten folgender Zusammensetzung ausgespatelt:

0,10 % Sojamehl

0,10 % Maisquellwasser

0,50 % Casein

0,20 % Gelatine

0,24 % $KH_2PO_4$

0,10 % $MgCl_2.6H_2O$

0,05 % $MnSO_4.4H_2O$

0,02 % $CaCl_2.2H_2O$

0,04 % Na-Desoxycholat

1,00 % Glukose

1,60 % Agar

pH        5,5

Rest      Wasser

Um Mutantenstämme mit deutlich erhöhter Proteasebildung sofort erkennen zu können, wurde dem Agarmedium zusätzlich der Protease-Inhibitor Pepstatin in Konzentrationen von 12,5 g/ml zugesetzt. Anstelle von Pepstatin können auch andere Inhibitoren für Carboxylproteasen zugefügt werden. Auch die Zusammensetzung des Caseinatagarmediums ist nicht kritisch.

Die Platten wurden 3-4 Tage bei 30°C bebrütet, bis die Einzelsporen zu deutlichen Kolonien ausgewachsen waren. Nur Kolonien mit verstärkter caseinolytischer Hofbildung wurden anschließend als Stammkulturen isoliert und weiter untersucht. Die Auswahlkriterien zur Isolierung leistungsfähiger Mutantenstämme waren durch die Zugabe des Protease-Inhibitors sehr verschärft und damit gewährleistet, daß nur Stämme mit signifikant erhöhter Proteasebildung selektiert wurden. Die neuen Stämme wurden zur Überprüfung ihrer proteolytischen Aktivitäten in Schüttelkulturen bei 30° C kultiviert (500 ml Erlenmeyerkolben mit Schikanen, Schüttelfrequenz 150 U/Min.). Die Zusammensetzung des Mediums war wie folgt:

$$
\begin{array}{ll}
0,30 \% & \text{Sojamehl} \\
0,30 \% & \text{Maisquellwasser} \\
1,00 \% & \text{Gelatine} \\
1,50 \% & \text{Casein} \\
0,24 \% & KH_2PO_4 \\
0,10 \% & MgSO.7H_2O \\
0,05 \% & MnCl_2.4H_2O \\
0,02 \% & CaCl_2.2H_2O \\
1,0 \% & \text{Maisstärke (amylolytisch abgebaut)} \\
pH & 5,5 \\
\text{Rest} & \text{Wasser.}
\end{array}
$$

Auf diese Weise wurden aus dem Wildstamm Mutantenstämme isoliert, die höhere Proteaseaktivitäten bilden. Von dem aktivsten dieser Stämme ausgehend wurden die Mutationsversuche fortgesetzt und nochmals eine Steigerung der
Syntheseleistungen erzielt. Die Mutationsserien wurden
insgesamt 3 mal durchgeführt und dabei folgende Stämme
isoliert:


T A B E L L E   1

| Stämme | Protease-Bildung (mTU/ml)AP 114 |
|---|---|
| AP 114  III -  69 (CBS 320.81) | 16,0 |
| AP 114 - IV  - 70 (CBS 321-81) | 27,7 |
| AP 114 - IV  - 74 (CBS 322.81) | 21,4 |
| AP 114 - IV  - 80 (CBS 323.81) | 21,6 |
| Aspergillus niger AP 114 (CBS 319.81) var. Tienhem (Wildstamm) | 8,0. |

Die von den Stämmen der Tabelle 1 gebildete Protease besitzt die gleichen Eigenschaften, wie diejenige des
Wildstamms CBS 319.81. Sie zeichnet sich durch ein besonders breites Wirkungsspektrum im schwach sauren Bereich zwischen pH 2,5 bis 6,5 aus. Das Wirkungs-Optimum
liegt bei pH 4,5, der Bereich der 80%igen Maximalaktivität bei pH 3,3 bis 5,9, der Bereich der 60%igen Maximalaktivität bei pH 3,0 bis 6,4.

Die Proteasebildung von anderen, saure Protease bildenden Aspergillus-Gattungen, wie Aspergillus phoenicii
(ATCC 14332), Aspergillus awamori (ATCC 14333), Aspergillus foetidus (ATCC 14334), Aspergillus awamori (ATCC

/6

14335) oder Aspergillus niger var. macro (ATCC 16513)
kann durch das neue Mutations- und Selektionsverfahren
auch erhöht werden.

Mit den genannten Aspergillus-Stämmen wurden folgende
Versuche durchgeführt.

1.) Bestimmung der Proteaseausbeuten im Schüttelkolben.

Die Ergebnisse sind in Tabelle 2 zusammengefaßt.

Tabelle 2

| Stämme | Protease-Bildung (m TU/ml) |
|---|---|
| 1. Aspergillus phoenicii ATCC 14332 | 9,0 |
| 2. Aspergillus awamori ATCC 14333 | 9,8 |
| 3. Aspergillus foetidus ATCC 14334 | 0,5 |
| 4. Aspergillus awamori ATCC 14335 | 13,1 |
| 5. Aspergillus niger ATCC 16513 var. macro | 7,2 |

2.) Charakterisierung durch Isoelektrofocussierung
    (vergl. Beispiel 4)
    Es zeigte sich, daß diese Stämme praktisch das
    gleiche extrazelluläre Protein- und Proteasespektrum
    aufweisen wie Aspergillus niger AP 114 und dessen
    Mutanten.

3.) Charakterisierung durch immunologische Verfahren
    (vergleiche Beispiel 4)
    Der Stamm Aspergillus niger AP 114 und seine Hochleistungsmutanten zeigen Kreuzreaktionen mit allen

/7

Proteasen der obengenannten Aspergillus Stämme,
nicht jedoch mit Proteasen anderer biologischer
Herkunft.

Die Protease eignet sich in besonderem Maße als Zusatzstoff für Tierfuttermittel, insbesondere zur Verbesserung der Ergebnisse bei der Aufzucht und Mast von Geflügel, Schweinen, Kälbern und Nutzfischen. Sie kann jedoch
darüber hinaus auch für andere Einsatzzwecke, bei denen
saure Proteasen verwendet werden können, angewendet werden, wie z.B. in Lebensmittel verarbeitenden Betrieben,
in sauren Wasch- und Reinigungsmitteln, insbesondere
Reinigungsmitteln für Kacheln, Fußböden und Tische, in
Krankenhäusern und im Haushalt, als Lederhilfsmittel in
der Gerberei, ferner in hoch gereinigter Form im medizinischen Anwendungsbereich als Digestivum.

Das Verfahren zur Herstellung der Protease kann durch
Züchten der selektierten Mutanten in einem flüssigen
oder festen Nährmedium durchgeführt werden, wobei das
flüssige Nährmedium im allgemeinen bevorzugt wird. Bei
Züchtung in einer Nährlösung wird nach dem üblichen
aeroben Schüttel-Kultur- oder Fermentationsverfahren
gearbeitet.

Der erfindungsgemäß zu verwendende Nährboden wird in üblicher Weise hergestellt und soll eine Kohlenstoffquelle, eine Stickstoffquelle und andere von dem Mikroorganismus benötigte Nähr- und Wuchsstoffe enthalten. Als
geeignete Kohlenstoffquellen sind Stärke, Dextrin, Rohrzucker, Glukose, Fruktose, Maltose und zuckerhaltige Abfallstoffe zu nennen. Als Stickstoffquellen kommen Ammoniumsalze, Harnstoff, Casein, Gelatine, Maisquellwasser
und Sojabohnenmehl bzw. -kuchen in Frage. Weiterhin können anorganische Salze, wie beispielsweise Natrium-, Kalium-, Ammoniumhydrogenphosphate, Calcium- und Magnesiumsalze dem Nährboden zugesetzt werden. Ferner kann es vorteilhaft sein, Wuchsstoffe, wie zum Beispiel Hefeextrakt und Vitamine dem Nährboden zuzusetzen.

Die Fermentationstemperatur kann sich in den Grenzen von 25 $^{\circ}$C bis 50 $^{\circ}$C bewegen, ist aber vorzugsweise zwischen 27 bis 32 $^{\circ}$C zu wählen. Der pH-Wert des Nährbodens kann 3,0 bis 7,0 betragen, vorzugsweise 4,0 bis 6,0. Die Züchtung wird im allgemeinen in einer Zeit von 20 bis 96 Stunden durchgeführt.

Die nach dem erfindungsgemäßen Verfahren hergestellte Protease kann aus der filtrierten oder zentrifugierten Nährlösung nach üblichen Methoden durch Zusatz organischer Lösungsmittel oder durch Aussalzen mit zum Beispiel Natriumsulfat oder Ammoniumsulfat ausgefällt und konzentriert werden. Eine Reinigung läßt sich durch Dialyse-Verfahren oder durch Behandlung an Ionenaustauscherharzen bewerkstelligen.

Die Feststellung der proteolytischen Aktivität der vorliegenden Protease erfolgte nach dem bekannten Prinzip der Bestimmung nach Anson: eine geeignet verdünnte Menge Enzymlösung wird bei 40 $^{\circ}$C 20 Minuten mit einem gleichen Volumen einer 1,2 %igen Caseinlösung inkubiert, wobei diese 0,6 % Milchsäure, 6 Mol Harnstoff und 0,1 Mol Zitronen- oder Essigsäure enthält. Der pH-Wert der Caseinlösung wird durch Zusatz von 2 N Natronlauge auf 4,5 eingestellt. Nach der Inkubation wird im Volumenverhältnis 1 : 1 mit 0,4 N Trichloressigsäure versetzt, der sich bildende Niederschlag von unverdautem Casein abfiltriert und im Filtrat die beim Abbau entstandenen Protein-Spaltstücke nach einer beliebigen Eiweißbestimmungsmethode ermitteln. Geeignet hierfür ist zum Beispiel das von Layne in Methods of Enzymology 3 (1957) Seiten 448 ff. beschriebene Verfahren.

Für jede Meßprobe muß ein Blindwert angefertigt werden, bei dem zuerst Trichloressigsäure und dann Caseinlösung zugesetzt werden. Dieser Blindwert gibt neben dem Reagenzien-Leerwert den Anteil an niedermolekularen Peptiden an, der bereits vor der Verdauung in der Enzymlösung vorhanden ist. Die Differenz zwischen Haupt- und Blindwert wird bei der angegebenen Methode dann mit der Extinktion verglichen, die eine bestimmte Menge Tyrosin bei dieser Bestimmung liefert. Diese Menge Tyrosin ist dann ein Maß für die proteolytische Aktivität des vorliegenden Enzyms: eine Enzymeinheit (TU) ist diejenige Menge Enzym, die den gleichen Extinktionsunterschied zwischen Haupt- und Blindwert pro Minute verursacht wie eine 1 M Tyrosinlösung, die anstatt der Enzymlösung eingesetzt wird.

Die Messung der proteolytischen Aktivität bei höheren und tieferen pH-Werten als 4,5 ist durch passende Einstellung der Caseinlösung ohne weiteres möglich, wobei man jedoch den Essigsäurezusatz günstigerweise durch Zitronensäure ersetzt.

## Beispiel 1

Zur Bereitung des Nährmediums wurden in 1 l Wasser 3 g Sojamehl, 3 g Maisquellwasser, 15 g Casein, 7 g Gelatine, 2,4 g $KH_2PO_4$, 0,5 g $MgSO_4$ . $7H_2O$, 0,1 g $MnCl_2$ . $4H_2O$, 0,1 g $CaCl_2$ . $2 H_2O$ und 20 g Maisstärke gelöst bzw. dispergiert. Der pH-Wert der Nährlösung betrug 5,3. Die Maisstärke wurde mittels Amylase weitestgehend abgebaut. In die sterilisierte Nährlösung wurden Sporen des Mutanten CBS 320.81 eingeimpft und die Kultur unter optimaler Belüftung bei 30° C etwa 50 Stunden im Schüttelinkubator gezüchtet. Nach dieser Zeit wurde das Pilzmycel abfiltriert und die mycelfreie Kulturbrühe zur Bestimmung der Proteaseaktivität gemäß vorgenanntem Verfahren verwendet. Dabei ergab sich, daß die Kulturlösung eine enzymatische Aktivität von 16.0 mTU/ml erreichte.

## Beispiel 2

Zur Bereitung des Nährmediums wurden in 1 l Leitungswasser 10 g Sojamehl (entölt), 5 g Maisquellwasser, 12 g Casein, 5 g Gelatine, 5 g Getreidetrockenschlempe, 2,4 g $KH_2PO_4$, 1 g $NaNO_3$, 1 g $NH_4Cl$, 0,01 g $FeSO_4$ und 30 g native Maisstärke gelöst bzw. dispergiert. Der pH-Wert der Nährlösung wurde nach dem Autoklavieren auf 5,3 eingestellt. Ein mit dieser Nährlösung angesetzter 1-Liter-Erlenmeyer-Kolben wurde mit 10 ml einer Czapek-Dox-Vorkultur (mit 5 % Stärke und 0,5 % Hefeextrakt) versetzt, die mit Sporen des Mutanten CBS 321.81 beimpft und 24 Stunden bei 30° C geschüttelt worden war, unter Belüftung bei 30° C etwa 72 - 96 Stunden betrieben, bis der pH-Wert auf 6,8 bis 7,0 angestiegen war. Danach wurde das Mycel abfiltriert und die klare Kulturbrühe zur Bestimmung der Proteaseaktivität gemäß vorgenanntem Verfahren verwendet. Dabei ergab sich, daß die Kulturlösung eine enzymatische Aktivität von 27.7 mTU/ml er-

/11

reichte. Die Protease wurde isoliert durch Klarfiltration der Brühe aus 10 l Schüttelkolbenansätzen, unter Zusatz von 5 g Filter Cel und 5 g Standard Super Cel der Firma Mansville, Einengen bei 50 Torr auf ein Drittel des Ausgangsvolumens und Ausfällen unter Zusatz von 39 % wasserfreiem Natriumsulfat unter Rühren, wobei die Temperatur auf 38 - 40° C gebracht wurde. Alternativ kann die Protease aber auch durch Zutropfen von 2 Volumina Ethanol, Methanol, Aceton oder anderer wassermischbarer Lösungsmittel bei einer Temperatur von -3 bis 5° C ausgefällt werden. Der Niederschlag wurde abgesaugt und im Vakuum getrocknet.

Beispiel 3

Unter Anwendung des vorstehend geschilderten Bestimmungsverfahrens wurde die Aktivität der nach Beispiel 1 und 2 isolierten Protease in Abhängigkeit vom pH-Wert ermittelt. In der Tabelle sind die pH-Werte aufgeführt, bei denen die optimale bzw. 50 % der beim optimalen pH-Wert gemessenen Aktivitäten vorliegen. Wie die Werte erkennen lassen, besitzen die Proteasen der in den Beispielen 1 und 2 verwendeten Mutanten den gleichen breiten und für die genannten Anwendungsgebiete günstigen pH-Wirkungsbereiche, wie die aus der Wildform CBS 319.81 isolierte Protease.

T A B E L L E 3

| Enzym | pH-Optimum | 50 %-Werte |
|---|---|---|
| Protease aus Mutante CBS 320.81 | 4,0 - 4,5 | 2,5 - 6,5 |
| Protease aus Mutante CBS 321.81 | 4,0 - 4,5 | 2,5 - 6,5 |
| Protease aus CBS 319.81 (Wildform) | 4,0 - 4,5 | 2,5 - 6,5 |

Beispiel 4

Die aus den Mutanten CBS 320.81, CBS 321.81, CBS 322.81, CBS 323.81 gewonnen Proteasen wurden nach der Methode der Isoelektrofokussierung bzw. der Kreuzreaktion nach Ouchterlony mit der aus dem Wildstamm CBS 319.81 gewonnenen Protease verglichen. Hierbei ergab sich praktisch vollständige Identität sämtlicher Proteasen.

Methodenbeschreibung Isoelectrofoccussierung

Geräte:     Multiphor 2117, LKB Instruments
            LKB 2103 Power Supply

Material:   LKB Ampholine PAG plates, LKB Instruments
            pH 3,5 - 9,5
            Anodenflüssigkeit : 1 M $H_3PO_4$
            Kathodenflüssigkeit : 1 M NaOH

Isoelectrofoccussierung:

Nach dem Aufbringen des Polyacrylamidgels auf die wassergekühlte Apparatur werden mit Elektrodenlösung getränkte Papierelektrodenstreifen auf die Ränder des Gels
gelegt. Auf der Mittellinie des Gels zwischen Anode und
Kathode werden 5 mm breite Filterstreifen aufgebracht
und 10 ul salzfreie Probelösung (5 mg/ml Protein) aufgetropft. Nach Verschließen der Apparatur wird bei einer
Stromstärke von 10 mA der pH-Gradient aufgebaut, nach 30
Min. die Probefilterstreifen entfernt und auf eine
Stromstärke von 15 mA erhöht. Nach 2 - 2,5 Stunden ist
die Trennung beendet.


Bestimmung der pH-Gradienten:

Der pH-Gradient wird durch eine kalibrierte OberflächenpH Elektrode dadurch bestimmt, daß man den pH-Verlauf
zwischen Anode und Kathode auf mehreren Linien abgreift
und ein pH/cm-Diagramm erstellt.


Fixierung und Färbung:

Fixierlösung:    Zu 57,5 g Trichloressigsäure und 17,25 g
                 Sulfosalicylsäure wird destilliertes
                 Wasser gegeben und auf 500 ml aufgefüllt.

Entfärbelösung: Eine Mischung aus 500 ml Ethanol und 160 ml Essigsäure wird mit destilliertem Wasser zu 2 000 ml verdünnt.

Färbelösung: 0,46 g Coomassie Blue G- 250 in 400 ml Entfärbelösung.

Nach der Erstellung des pH-Profils wird das Gel 1 Stunde zur Proteinpräzipitation und Ampholin-Entfernung in gerührter Fixierlösung belassen, 5 Minuten mit Entfärbelösung gewaschen und anschließend für 10 Minuten bei 60 $^{\circ}$C in die Färbelösung gelegt. Das Gel wird durch mehrmaliges Wechseln der Entfärbelösung entfärbt, bis sich die blaugefärbten Proteinbanden deutlich vom Untergrund abheben.

Anhand des pH-Profils werden die isoelektrischen Punkte der Proteine bestimmt.

Methodenbeschreibung der Kreuzreaktion nach Ouchterlony

Material

Für die Kreuzreaktionen werden Immundiffusionsplatten der Firma Miles, Frankfurt eingesetzt (Code Nr. 64-276-1). Die Fertigplatten enthalten 0,9 %ige Agarose in Borat/Kochsalzpuffer, pH 7,5 - Ionenstärke 0,175 %. Die Platten enthalten ferner 0,01 % Thiomersal als Bakteriostatikum und Trypanblau als Indikator für die proteinpräzipitationslinien.

Antikörper gegen die Protease CBS 319.81 wurden aus Kaninchen gewonnen.

Als positive Kontrolle diente Protease CBS 319.81 mit der die Immunisierung durchgeführt wurde.

Durchführung

In der Mitte der Diffusionsplatten werden 25 ul des Antiserums hineinpipettiert, in die außen gelegenen Löcher jeweils 25 ul Proteaselösung bei einer Konzentration von 5 mg/ml bzw. 10 mg/ml.

Diffusionszeit: 72 Stunden bei 4 °C.

Präzipitationslinien können durch den in der Agarose enthaltenen Trypanblauindikator nachgewiesen werden. Die immunologische Kreuzreaktion fällt nur dann positiv aus, wenn die Antikörper erzeugende Protease mit der geprüften Protease identisch ist. Dies wird durch Verschmelzen der Präzipitationslinien festgestellt.

Tabelle 4

Ergebnisse der isoelektrischen Focussierung

| PI-Werte | ATCC 14332 | | ATCC 14333 | | ATCC 14334 | | ATCC 14335 | | ATCC 16513 | | CBS 320.81 | |
|---|---|---|---|---|---|---|---|---|---|---|---|---|
| | a | b | a | b | a | b | a | b | a | b | a | b |
| 3,5 | xxxx | - | xxxx | xx | xxxx | xx | xxxx | xx | xxxx | x | xxxx | xxx |
| 3,7 | x | - | xx | xx | xx | xx | xx | xx | - | - | xx | x |
| 3,95 | x | - | - | - | - | - | - | - | - | - | - | - |
| 4,0 | x | xxxx | xxxx | xxxx | xxxx | xxxx | xxxx | xxxx | xxx | xxx | xxxx | xxxx |
| 4,05 | x | - | x | - | x | - | x | - | - | - | x | - |
| 4,3 | x | - | x | - | x | - | x | - | - | - | x | - |
| 4,6 | x | - | x | - | x | - | x | - | - | - | x | - |

a) Coomassienblue    (= Protein)

b) Zymogramm    (= Protease)

Patentanmeldung D 5958 EP - 16 -

HENKEL KGaA
ZR-FE/Patente

0082395

## Tabelle 4   Fortsetzung

| PI-Werte | CBS 321.81 | | CBS 322.81 | | CBS 323.81 | | CBS 319.81 | |
|---|---|---|---|---|---|---|---|---|
| | a | b | a | b | a | b | a | b |
| 3,5 | xxxx | xx | xxxx | xx | xxxx | xx | xxxx | xx |
| 3,7 | xx | xx | xx | xx | xx | xx | xx | xx |
| 3.95 | – | – | – | – | – | – | – | – |
| 4,0 | xxxx | xxxx | xxxx | xxxx | xxxx | xxxx | xxxx | xxxx |
| 4,05 | x | – | x | – | x | – | x | – |
| 4,3 | x | – | x | – | x | – | x | – |
| 4,6 | x | – | x | – | x | – | x | – |

0082395

HENKEL KGaA
ZR-FE/Patente

## Tabelle 5

| Protease | Kreuz-reaktio-nen gegen AP 114 - Antikörper | Kreuz-reaktionen gegen SP 1 - Antikörper |
|---|:---:|:---:|
| 1. Aspergillus phoenicii ATCC 14332 | + | − |
| 2. Aspergillus awamori ATCC 14333 | + | − |
| 3. Aspergillus foetidus ATCC 14334 | + | − |
| 4. Aspergillus awamori ATCC 14335 | + | − |
| 5. Aspergillus niger ATCC 16513 var. macro | + | − |
| 6. AP 114 - III - (69 CBS 320 81) | + | − |
| 7. AP 114 - IV - (70 CBS 321 81) | + | − |
| 8. AP 114 - IV - (74 CBS 322 81) | + | − |
| 9. AP 114 - IV - (80 CBS 323 81) | + | − |
| 10. Aspergillus niger AP 114 var. Tienhem (CBS 310 81) | + | − |

Patentansprüche

1. Verfahren zur Herstellung von saurer Protease mit
   breitem pH- Wirkungsbereich in hoher Ausbeute durch
   aerobes Züchten eines Pilzes der Gattung Aspergillus,
   dadurch gekennzeichnet, daß man
   a) einen Wildstamm der Gattung Aspergillus, welcher
   saure Protease bildet, isoliert und
   b) einer Mutierung, vorzugsweise durch UV-Bestrahlung, unterwirft und
   c) Mutanten mit einer erhöhten Proteasebildung von
   über 15 mTU/ml selektiert, indem man sie auf Caseinatagarplatten ausspatelt, dem Agar-Medium einen
   Inhibitor für Carboxylprotease, vorzugsweise Pepstatin, zugibt, einige Tage bebrütet und die Mutantenkolonien mit verstärkter caseinolytischer
   Hofbildung isoliert und
   d) diese Mutanten in einem Nährmedium, das assimilierbare Kohlenstoff- und Stickstoffquellen enthält,
   bei pH-Bereichen zwischen 3 bis 7 und Temperaturen
   zwischen25 und 50 $^\circ$C züchtet und
   e) die gebildete Protease abtrennt.

2. Verfahren zur Herstellung von saurer Protease nach
   Anspruch 1,dadurch gekennzeichnet, daß man einen
   Pilzstamm der GattungAspergillus niger verwendet.

3. Verfahren zur Herstellung saurer Proteasen nach Anspruch 1 bis 2, dadurch gekennzeichnet, daß man den
   Wildstamm Aspergillus niger var. Tienheim CBS 319.81
   verwendet.

4. Verfahren zur Herstellung saurer Proteasen nach Anspruch 1 bis 3, dadurch gekennzeichnet, daß man das Mutierungs- und Selektionsverfahren ein- oder mehrfach wiederholt.

5. Mutanten des Wildstammes Aspergillus niger var. Tienhem CBS 319.81, die in Schüttelkulturen eine proteolytische Aktivität von mehr als 15 mTU/ml bilden, und deren Protease mit derjenigen des Wildstammes identisch ist.

6. Mutanten des Wildstammes Aspergillus niger var. Tienhem CBS 319.81 mit der Hinterlegungsbezeichnung

  Aspergillus niger AP 114 - III - 69  (CBS 320.81)
  Aspergillus niger AP 114 - IV  - 70  (CBS 321.81)
  Aspergillus niger AP 114 - IV  - 74  (CBS 322.81)
  Aspergillus niger AP 114 - IV  - 80  (CBS 323.81)

7. Wildstamm Aspergillus niger var. Tienhem mit der Hinterlegungsbezeichnung AP 114 (CBS 319.81).

8. Verwendung der nach Anspruch 1 - 4 hergestellten Protease als Tierfutterzusatz.